# EUROPEAN PATENT APPLICATION

(11) **EP 3 916 382 A1**
(43) Date of publication of application: **01.12.2021**
(21) Application number: 20176614.4
(22) Date of filing: 26.05.2020
(51) Int. Cl.: G01N 29/00, G01N 33/543

(54) **DETECTION OF PATHOGENS AND INFECTIONS BY MASS-SENSITIVE SENSOR MICRO-ARRAYS**

(71) Applicant: BioMensio Ltd., 33720 Tampere (FI)
(72) Inventor: AUER, Sanna, 33960 Pirkkala (FI); FREYTAG, Rudolf, 85598 Baldham (DE); BUCHHOLZ, Stephan, 81739 München (DE); SPEHAR, Anna-Maria, 33540 Tampere (FI); TÖRMÄLÄ, Sauli, 02230 Espoo (FI); SCHREITER, Matthias, 81379 München (DE)
(74) Representative: Kolster Oy Ab

(57) **Abstract**

The present invention relates to a device (100) for detecting at least one substance (SB, SB1, SB2) of a sample (SA1, SA2). The device (100) comprises a mass sensitive micro-array (MSMS) (10) with a plurality of mass sensitive detector elements (RE, RE1, RE2) each comprising a detection layer (L, L1, L2) with a surface section (S, S1, S2). The detection layer (L, L1, L2) of at least one of the detector elements (RE, RE1, RE2) is activated for sorption of the at least one substance (SB, SB1, SB2) of the sample (SA1, SA2), wherein the sample (SA1, SA2) is preferably obtained from a subject. Further, the present invention is directed to a method for the analysis of at least one substance (SB, SB1, SB2) in a sample (SA1, SA2) providing the device (100). Also, the present invention relates to a detector kit (101) comprising the device (100) and a control system (CD) comprising a processor, a memory and a program to analyse the at least one substance (SB, SB1, SB2).

## Description

The present invention relates to the detection of pathogens and infections, namely to a device for detecting of at least one substance of a sample, comprising a mass sensitive micro-array. Further, the present invention is directed to a method for the analysis of at least one substance in a sample providing the device. Also, the present invention relates to a detector kit comprising the device.

Contaminants and pathogens are currently inexorable making them a major issue in global safety, particularly those that pose serious health concerns to humans and animals. A lot of them are of low-molecular weight substances. The difficulties associated with remediation combined with the health risks of contaminants and pathogens has provoked control methods to ensure safety of humans and animals. This includes utilising accurate and reliable testing methods to prevent infections and diseases caused by pathogens.

The detection of contaminant molecules has been ongoing for approximately 50 years. This has subsequently driven the development and commercializing of methods for molecular analysis of infections and pathogens. A lot of methods for analysis are available and their usage is dependent on a variety of factors. For example, liquid chromatography tandem mass spectrometry (LC-MS/MS) is extensively used as a confirmatory reference method as it allows the simultaneous determination of different molecules. Other chromatographic-based methods exist which can be coupled with ultraviolet (UV), diode array (DAD), and fluorescence (FLD) detectors including thin-layer chromatography or high-performance liquid chromatography (HPLC). These methods, however, are limited to large commercial companies, reference and academic laboratories with skilled technicians and expensive laboratory equipment. Furthermore, the methods are generally time-consuming and labour intensive.

In this regard, rapid commercial test kits for analysis are used as an alternative for more user-friendly and robust detection. The majority of test kits on the market today are based on an immunoassay format which relies on the ability of an antibody to bind to a specific antigen structure (target molecule). For the detection of small molecules (less than 1 kDa) a 'sandwich' type immunoassay in which multiple antibodies bind to a single molecule is not suitable, rather a competitive assay is generally employed. In a competitive immunoassay, the analyte of interest from a sample will affect the detection and measurement of a competitor, meaning the result generated is an indirect measurement of the analyte of interest. The properties of the competitor will vary depending on the assay but regardless, every competitor must be capable of triggering a signal that can be detected and measured by the device/reader in place for that assay. Conventional and currently manufactured immunoassay-based test kits for screening of molecules include Enzyme-Linked Immuno-Sorbent Assays (ELISAs), Lateral Flow Immunoassays (LFIA) and fluorometric assays.

Assay requirements are evolving which has an impact on the performance of the current above-mentioned immunological test kits. The main current trends to be met include portability and multi-molecular detection. The reason for portability becoming increasingly important lies in the growing demand for on-site testing. On-site testing can occur at points in the health area. For example, at a medical's practice analysis of the material can be conducted in a low technology environment using a portable test kit. Results can be obtained rapidly as samples are not required to be sent off to reference laboratories for analysis. This also prevents the therapy process slowing down. On-site testing can therefore be considered a time efficient and cost-effective choice. Therefore, portable multi-molecular testing devices suitable for on-site testing would give added cost- and time-saving benefits. This holds even more, if the test is supposed to provide data at a good sensitivity without the need of additional steps such as, e. g. labelling of one or more substances.

It is therefore an object of the invention to provide an improved equipment and method for time-efficient on-site detection of a substance in a sample exhibiting good sensitivity and reliability without the need of labelling such a substance.

This object is achieved by the device according to claim 1, a method for the analysis of at least one substance according to claim 13 and a detector kit according to claim 15.

According to the invention, the device for detecting at least one substance of a sample, preferably in a liquid or liquefied, comprises a mass sensitive micro-array (in the following also abbreviated as "MSMA") with a plurality of mass sensitive detector elements each comprising a detection layer with a surface section.

According to the invention, the detection layer of at least one of the mass sensitive detector elements is activated for sorption of the at least one substance of the sample, wherein the sample is preferably obtained from a subject, more preferably a human or an animal, even more preferably a human, in particular wherein the sample is chosen from one or more of (whole) blood, plasma, serum, saliva, cerebrospinal fluid, sweat, tears, faeces, vomit, nasopharyngeal swab, tracheal aspirate and/or urine.

In other words, the particular mass sensitive detector element exhibits (due to the activation) an activated detection layer, which is prepared (activated) in such a way that the mass sensitive detector element is capable to detect the substance contained in the sample. Principles of activation are explained below.

According to the invention, "mass sensitive detector elements" as used herein refer to elements of the mass sensitive micro-array wherein each of the elements in the micro-array may serve to detect a specific substance. The term "mass sensitive detector element" as used herein is synonymously named "detector element" or "pixel" of the mass sensitive micro-array. Typically, the size of such a mass sensitive detector element is in the range of several tens to several hundreds of µm.

In a particularly preferred device, a detector element of the MSMA comprises at least one resonator element (herein also referred to as "resonator") as a "transducer" to transduce the mass change due to a sorption of a substance on the detection layer of the detector element into an electrical signal.

The detection layer - including the activated surface section - is integrated within the mass sensitive detector element (preferably as a top layer) so that upon interaction with a sample, a binding event or reaction occurring on the detection layer will generate an electronic signal via the detector element (by using the mass sensitivity of the detector element) that is measured by a control system as explained below. Such an electronic (output) signal represents an (output) value, which comprises the information of the existence and/or amount of the substance at the surface section as explained below.

The surface section of the detection layer (which may be the complete surface or just a part of the surface of the detection layer) is typically activated, i. e. prepared or coated to comprise one or more detection agents, such as biological material, a biologically derived material and/or a biomimetic. Preferred detection agents, which can be used for the detection, are disclosed elsewhere herein.

In order to improve binding of the detection agent, the surface may be particularly prepared, e. g. cleaned or precoated, prior to activation.

The surface of the detection layer, preferably, comprises said detection agents in immobilized form on a surface which is to be contacted with the sample comprising the at least one substance, the amount of which is to be determined.

Preferably, the detection layer comprises a surface coating, capable to determine the amount of the at least one substance, which is specifically bound to the at least one detection agent. In particular, the detection layer allows for quantitative detection of the at least one substance.

As mentioned above, according to the invention, the activation of the detection layer of a detector elements is such that at least one substance of the sample, which may preferably obtained from a subject, is sorbed.

Within the context of the present invention, the term "sorption" means the formation of a chemical or physical bonding of the substance to the surface section and includes both absorption and adsorption.

The term "sample" as used herein refers to a sample of a subject, preferably of a body fluid, more preferably to a sample of blood (including whole blood), plasma or serum. The term, however, also encompasses samples, which are derived from, e. g. one or more of(whole) blood, plasma, serum, saliva, cerebrospinal fluid, sweat, tears, faeces, vomit, nasopharyngeal swab, tracheal aspirate and/or urine. The sample may be treated by, e.g. pre-treatment steps such as preparing fractions of, e. g. blood, plasma or serum obtained by, e.g. one or more purification step/s. Preferably, the sample is a liquid.

The term "subject" as used herein refers to an animal, preferably, a mammal and, most preferably, a human. The subject may be in need of disease diagnostic and disease therapy, preferably wherein the disease is a result from bacterial and/or viral infection.

Since each of the detector elements of the mass sensitive micro-array serve to detect at least one specific substance, it is in principle possible to allow for multi-molecular testing without the need to purchase and run multiple single-molecule tests for each sample, thereby generating one or more result/s at good sensitivity and time-efficient manner.

Though, by using the device according to the invention it is in principle possible to detect substances comprising a label. Beneficially, the advantageous device allows for detection of substances without labelling. By using such a non-labelled substance, any sterical effect impacting on the binding of a labelled substance to a substrate is avoided, thereby even allowing for real time measurement of binding kinetics of a specific substance to a certain substrate.

Preferably, based on the plurality of detector elements of an MSMA, it is advantageously possible to activate a plurality of detector elements to sorb the same substance of the sample therefore allowing for a redundant measurement of the substance. By generating a (statistically relevant) number of redundant data, a statistical analysis is possible, such as statistical analysis of different virus samples comprising, e. g. different virus strains, e. g. different strains of SARS-CoV-2.

Advantageously, by applying the device of the invention, in contrast to the visual read-out for the majority of other assays, such data are obtained in digital format. The digital data may be further transmitted to a central database without further efforts. This is of beneficial help in on-site testing.

Further, the present invention relates to a method for the analysis of at least one substance in a sample, preferably in a liquid, comprising the steps of
(i) providing a mass sensitive micro-array with a plurality of mass sensitive detector elements each comprising a detection layer with a surface section,
(ii) activating the detection layer of at least one of the detector elements for sorption of the at least one substance of the sample,
(iii) applying the sample onto the mass sensitive detector elements.

With regard to steps (ii) and (iii) the sample is preferably obtained from a subject, more preferably a human or an animal, even more preferably a human, in particular wherein the sample is chosen from one or more of(whole) blood, plasma, serum, saliva, cerebrospinal fluid, sweat, tears, faeces, vomit, nasopharyngeal swab, tracheal aspirate and/or urine.

In accordance with the present invention, the method is preferably an ex vivo and/or an in vitro method.

Preferably, the device and the method according to the invention allows not only to simply detect the existence of the substance but also for quantitative detection of the substance in the sample based on the use of the mass sensitive detector elements.

The amount determined by the mass sensitive detector element is represented by the electronic (output) signal of the mass sensitive detector elements which is transferred to the control system, as already mentioned.

Such a control system may comprise means for providing electrical power to the device, and means for recognising an electrical (output) signal of the detector elements, e. g. for monitoring the resonance frequency of the detector elements and to register any change in the resonance frequency as an electronic (output) signal.

Preferably, the control system may also comprise an evaluation unit to convert the electronic signals into suitable output values or "sample values", which, in turn, may be output to a user and/or used in any other way, e. g. stored in a memory or further processed.

Said evaluation unit may comprise a data processing element, such as a computer, in particular a micro-computer, preferably with an implemented algorithm for carrying out a comparison analysis, i. e. comparison of different sample values or comparison of one or more sample values with one or more reference values.

Accordingly, the method further comprises the steps of
(iv) obtaining at least one sample value from a mass sensitive detector element,
(v) quantifying the at least one sample value,
(vi) optionally comparing the at least one sample value with an at least one reference value.

E. g. based on the results of the comparison, the evaluation unit may "evaluate" whether a sample of a subject comprises at least one substance or not. Reference values may be obtained by other detector elements prior to, in parallel or after the measurement. Those detector elements may be prepared to serve as "reference detector elements". Additionally or alternatively, reference values may also be taken from a database.

Advantageously, the reference values are to be chosen so that either a difference or a similarity in the compared amounts allows for identifying the test subjects, which belong into the group of subjects, e. g. comprising or being infected with a substance (positive group) or not comprising or being infected with a substance (negative group).

For example, at least one of the detector elements of an MSMA may be configured to generate a reference value which may result, e. g. from a positive control or negative control, i. e. from a sample to comprise a substance known to bind to a specific detection agent (positive control) or from a sample known to lack the specific substance and/or where the surface of the detection element does not comprise a specific detection agent (negative control).

The comparison with reference values will also be described in more detail below.

The results, comprising the sample values and/or results of analysis of these sample values or reference values may be given as output of diagnostic (raw) data, preferably, as absolute or relative amounts. It may be suitable that these data may need interpretation by a clinician. However, also envisaged are devices and methods wherein the output comprises processed diagnostic raw data the interpretation of which does not require a specialized clinician.

The control system may preferably further comprise a user interface such as a display and an input unit (preferably a touch screen). E. g. a smart phone (with an app and a suitable interface to the device, such as an interface coupled to the smart phone via Bluetooth, USB, etc.) may be used for such a purpose. For example, a physical interface for coupling with the device may be a "box" which takes in the device (in form of a cartridge) and is coupled to the smartphone per wire or wirelessly.

The control system may, at least partially, be integrated on the device. In particular, the evaluation unit may be part of the device itself.

The control system may, at least partially, be externally and the device and the control system may comprise suitable interfaces for coupling the device and the control system. The control system may comprise several sub-units such as a computer and a reader unit as an interface for coupling the device with the computer.

The control system may, also preferably, comprise a compact control unit which may be enclosed in a portable housing (handheld unit) and which may comprise the user interface and the interface or reader unit for coupling with the device, e. g. in form of a slot in the housing in which the device may be inserted such that electrical contact elements of the device come in contact with electrical contact elements of the control unit.

Therefore, the present invention is also directed to a detector kit comprising at least one device according to the present invention preferably comprising a control system as explained above.

The control system comprises, as already mentioned, an evaluation unit which is constructed such that it quantifies at least one sample value obtained from a mass sensitive detector element and optionally compares the at least one sample value with an at least one reference value. The reference value may be, e. g. a baseline value.

Alternatively or additionally, the detector kit may comprise detection agents for detecting in a sample of the subject the presence, preferably the amount, of the at least one substance, particularly preferably the kit comprises detection agents for detecting in a sample of a subject the presence of a virus, mostly preferred the presence of a corona virus.

The sub-units or modules of the above-mentioned control system or control unit can be completely or partially realised as software modules running on a processor of the control unit. A realisation largely in the form of software modules can have the advantage that applications already installed on an existing system can be updated, with relatively little effort, to install and run the device of the present application.

Therefore, the present invention may also be directed to a computer program product with a computer program that is directly loadable into the memory of a control system, and which comprises program units to perform parts of the steps of the method according to the invention when the program is executed by the control unit, namely at least the steps which have to be done by the evaluation unit. In addition to the computer program, such a computer program product can also comprise further parts such as documentation and/or additional components, also hardware components such as a hardware key ((personalized) smartcard etc.) to facilitate access to the software.

A computer readable medium such as a memory stick, a hard-disk or other transportable or permanently-installed carrier can serve to transport and/or to store the executable parts of the computer program product so that these can be read from a processor unit of a control system.

Also, the device might have an internet connection to transfer results or even use a cloud based software for analysis of the results (e. g. using anonymized data for self-learning Artifical Intelligence based on the results of thousands of measured subjects, i. e. patients).

The comparison analysis referred to in the present invention may be carried out computer assisted as explained above. However, they may also be carried out manually.

Further preferred embodiments of the invention are derived from the dependent claims together with the following description, whereby the patent claims of a certain category may be formed by dependent claims of a different category, and features of the different examples may be combined to new examples. It is to be understood that the definitions and explanations of the terms made above and below apply accordingly for all embodiments described in this specification and the accompanying claims. In the following, particular embodiments of the method of the present invention are specified further.

According to a preferred embodiment, the mass sensitive micro-array comprises a resonator array with a plurality of film bulk acoustic resonator elements, preferably implemented as shear-mode operating solidly mounted resonator elements (in the following also referred to as "FBAR"). Such film bulk acoustic resonator elements are mass-sensitive sensors based on electroacoustic technology having a detection layer, comprising at least one thin polycrystalline piezoelectric layer with an electrode disposed on the polycrystalline piezoelectric layer and one or more further electrode/s disposed on the polycrystalline piezoelectric layer.

The construction and controlling of suitable embodiments of such a FBAR is described e. g. in WO 2004/017063 A1, which is, with regard to those aspects, incorporated herein by reference.

Preferably, the polycrystalline piezoelectric, the electrodes and the surface section of the detection layer are arranged with respect to one another in such a way that an electrical actuation of the electrodes leads to an oscillation of the resonator at a resonance frequency, wherein the resonance frequency is dependent on an amount of the substance sorbed on the surface section. The detection layer is integrated within the resonator element so that upon interaction with the sample, a binding event or reaction occurring at the detection layer will generate an electronic signal.

Advantageously, a layer thickness of the polycrystalline piezoelectric layer is chosen from at least 0.1 µm and/or up to 20 µm.

Preferably, the resonance frequency of the oscillation ranges from at least 500 MHz and/or up to 10 GHz. In particular, the oscillation of the resonator is thickness shear mode oscillation.

A preferred resonator may comprise a piezoelectric material, preferably a thin film at a thickness of at least 0.5 µm and/or up to 3 µm.

More preferably such a thin film comprises ferroelectrics such as lead-zirconate-titanate (Pb(ZrₓTi₁₋ₓ)O₃), and/or piezoelectrics such as aluminium-nitride (AlN) and/or zinc oxide (ZnO). Even more preferably, a thin piezoelectric film comprises aluminium nitride and/or zinc oxide.

Advantageously, aluminium nitride exhibits high values of chemical inertness, acoustic longitudinal wave velocity, electrical resistivity and superior piezoelectric behaviour.

As the resonator of such thin piezoelectric films operates at higher frequencies, the device can provide mass data of high sensitivity.

According to a preferred embodiment a resonator has a lateral extension of at least 20 µm and/or up to 1000 µm.

Advantageously, according to a preferred embodiment the resonator allows for detection of substances at a size of up to 100 000 kDa, more preferably up to 1 000 kDa, even more preferably up to 500 kDa, in particular up to 200 kDa, mostly preferred from 100 up to 150 kDa.

In particular, the resonator allows for detection of substances at a preferred size of about 10 nm and/or about 500 nm, more preferable about 20 nm and/or about 250 nm, even more preferable 50 nm and/or about 200 nm, in particular about 100 nm and/or about 150 nm.

In a further preferred embodiment the resonator is disposed on a semiconductor substrate.

Even more preferably, the device provides acoustic insulation of the resonator and the semiconductor substrate.

According to a preferred embodiment, each of the resonators in the mass sensitive micro-array serves to detect at least one specific substance. Particularly preferable, each of the resonators serve to detect exactly one specific substance.

A particularly preferred device comprises an evaluation unit, wherein the mass sensitive detector elements are connected to the evaluation unit to transfer at least one sample value to the evaluation unit and wherein the evaluation unit is constructed such that it quantifies the at least one sample value and optionally compares the at least one sample value with an at least one reference value (as already described above). In this embodiment the evaluation unit is integrated or placed on board of the device, in particular on the same circuit board or semiconductor substrate as the MSMA.

As already mentioned above the sample values may be compared with reference values to obtain a reliable result of a substance analysis, connected e. g. to a clinical aspect.

As used herein, the term "comparison" or "comparing" encompasses comparing the amount of the one or more substance/s comprised in the sample to be analysed with an amount of a suitable reference specified below.

It is to be understood that comparing as used herein refers to a comparison of corresponding parameters or values, e.g., an absolute amount is compared to an absolute reference amount while a concentration is compared to a reference concentration or an intensity signal obtained from a test sample is compared to the same type of intensity signal of a reference sample.

However, in accordance with the invention it is also envisaged to calculate a value based on the quantitative measurement of the substance. Further details may be found in the accompanying examples (below).

The term "reference" as used herein refers to a value or threshold based on amounts of the substance which allows for allocation of a sample (of a subject), e. g. to belong into the group of subjects comprising or being infected with a substance (positive group) or not comprising or being infected with a substance (negative group). Preferably, such a reference is a negative and/or positive control type or an individual reference.

Such a reference can be a threshold amount, which separates the groups (either positive or negative) from each other. A suitable threshold amount separating the two groups can be calculated without further ado by a test based on the amounts of the substance from either a subject or group of subjects known to belong to a positive or a negative group, e. g. exhibit a positive signal or a negative signal.

A reference can, in principle, also be calculated for a subject as specified above based on the average or mean values for a given substance by applying standard statistically methods. Accordingly, the reference to be used for the aforementioned device and/or method may be generated for said subject as described above and deriving a threshold amount therefrom.

Suitable references can be derived from samples to be used for the generation of a reference or reference amounts as described elsewhere herein above. The results may be given as output of diagnostic raw data, preferably, as absolute or relative amounts. It may be suitable that these data may need interpretation by a clinician. However, also envisaged are expert system devices wherein the output comprises processed diagnostic raw data the interpretation of which does not require a specialised clinician.

According to a preferred embodiment, the surface or a section of a surface of the detection layer of an advantageous device is coated with a surface coating comprising a detection agent, i. e. a substrate to comprise, i. e. a molecule capable to specifically bind the substance to be detected.

Preferably such a substrate comprises biological material such as, e. g. tissue, microorganisms, organelles, cell receptors, enzymes, antibodies, nucleic acids, natural products etc. Alternative or additionally such a substrate comprises biologically derived material such as, e. g. recombinant antibodies, engineered proteins, aptamers and/or a biomimetic such as, e. g. synthetic receptors, biomimetic catalysts, combinatorial ligands, imprinted polymers etc.

According to a preferred embodiment, the surface coating of the detection layer comprises a detection agent selected from
- at least one protein
   and/or
- at least one engineered protein,
   and/or
- at least one antibody and/or part/s of an at least one antibody, wherein the at least one antibody and/or part/s of an antibody are preferably chosen to bind to at least one pathogen and/or at least one protein of the at least one pathogen and/or at least one antigen of the at least one pathogen and/or at least one epitope of the at least one pathogen and/or at least one toxin derived from the at least one pathogen,
   and/or
- at least one aptamer,
   and/or
   at least one biomimetic.

An at least one protein for detection of a substrate may be either selected from a natural protein, e. g. a protein from a natural source. Alternative or in addition, a protein may be selected from an engineered protein, i. e. a protein derived from chemical synthesis which may comprise one or more non-natural amino acids.

An at least one preferred antibody and/or part/s of an at least one antibody might be selected from a recombinant or engineered antibody, a single chain antibody, an Fab fragment of antibody and/or an (single) arm or part of an arm of an antibody.

The at least one antibody and/or part/s of an antibody are preferably chosen to bind to at least one pathogen and/or at least one protein of the at least one pathogen and/or at least one antigen, in particular an epitope, of the at least one pathogen and/or at least one toxin derived from the at least one pathogen.

Further preferably, the at least one antibody or part/s of an antibody is/are capable to bind to an antigen, which is, in particular, part of an antibody comprised in a sample as detailed above. Preferably, such a sample is derived from (whole) blood or serum. Even more preferably, such an antibody is an IgG antibody and/or an IgM antibody.

Beneficially, by measuring both IgG antibody and IgM antibody, it is possible to obtain information on a specific disease phase, i. e. an early phase of disease status to fight against a specific infection, which is indicated by an increased level of IgM antibodies, whereas an increased level of IgG antibodies indicates immunity against infection at a later stage.

This is preferably of importance when measuring the disease status after infection with a virus or a bacterium, more preferably a disease status resulting from an infection with a virus, even more preferably a coronavirus, in particular an infection resulting from SARS-CoV-2.

Preferably, an IgG antibody is selected from an IgG1 antibody, an IgG2 antibody, an IgG3 antibody and/or an IgG4 antibody.

According to a preferred embodiment, a detection agent comprised in the substrate coating comprises at least one aptamer. The term "aptamer" as used herein refers to an oligonucleotide or peptide molecule that binds to a specific substance (molecule), i. e. a substance to be analysed (i. e. the analyte) and comprised in the sample. Preferably, an aptamer comprises single-stranded oligonucleotides of DNA and/or RNA (with a length of, e. g. 10 to 100 nucleotides), capable of interacting with the substance. Advantageously, aptamers, unlike antibodies, can be regenerated and reused for other or further analyses, respectively.

Alternatively of additionally, a detection agent comprised in the substrate is selected from at least one biomimetic such as, e. g. a synthetic receptor, a biomimetic catalyst, a combinatorial ligand and/or an imprinted polymer.

According to a specific embodiment, the surface coating, i. e. the detection agent, comprises at least one protein and/or engineered protein chosen from at least one antibody or antibody fragment against at least one viral epitope or spike protein and/or at least one membrane protein and/or at least one nucleocapside protein and/or at least one ACE2 receptor protein. Preferrably, such a spike protein and/or membrane protein and/or nucleocapside protein and/or ACE2 receptor protein comprises a His-tag.

Immobilisation of such detection agents on the surface of the detection layer may be improved by pre-coating the surface of the detection layer. Such a pre-coating is known to the person skilled in the art and is described, e. g. in Vikholm-Lundin, I. et al. Sensors and Actuators B 171-172 (2012) 440-448.

According to a further preferred embodiment, the substance to be analysed, i. e. the analyte, is selected from one or more proteins, peptides, sugar moieties, DNA, RNA, virus/es or parts of virus/es, virus-like-particles, bacteria or parts of bacteria, bacteria-like-particles, toxin/s, metabolites. The DNA or RNA may be either single or double stranded. Preferably, the DNA or RNA is single stranded.

Even more preferably, the substance is selected from single stranded RNA, most preferably derived from one or more virus/es, which are, even more preferably, obtained from a sample to comprise saliva.

Further preferably, the substance is selected from antibodies which are, even more preferably, obtained from a blood or a serum sample.

According to a preferred embodiment, the substance is selected from a virus and/or part of a virus and/or from a bacterium and/or part of a bacterium.

According to a particularly preferred embodiment, the substance is selected from at least one virus or part of at least one virus, wherein the at least one virus is preferably selected from an influenza virus, in particular from influenza A virus; from a respiratory syncytial (RS)-virus; from a gastrointestinal virus, in particular from rotavirus, norovirus, adenovirus or astrovirus; from a respiratory virus, in particular from human bocavirus 1; from a coronavirus, in particular from HCoV-HKU1 (human coronavirus HKU1), HCoV-NL63 (human coronavirus NL63), HCoV-OC43 (human coronavirus OC43), HCoV-229E (human coronavirus 229E), SARS-CoV (severe acute respiratory syndrome-related coronavirus), MERS-CoV (middle East respiratory syndrome-related coronavirus) and/or SARS-CoV-2 (severe acute respiratory syndrome-related coronavirus 2).

In case the substance is derived from a bacterium or part of a bacterium, such a bacterium or part of a bacterium may be selected from, e. g. Streptococcus, such as Streptococcus A and/or Streptococcus pneumoniae; from Mycoplasma such as Mycoplasma pneumoniae; from Klebsiella such as Klebsiella pneumoniae; from Enterobacter such as Enterobacter agglomerans; from Haemophilus such as Haemophilus influenzae; from Chlamydophila such as Chlamydophila pneumoniae; from Coxiella such as Coxiella burnetii; from Legionella such as Legionella pneumophila; from Staphylococcus, such as Staphylococcus aureus; from Bacillus such as Bacillus cereus; from Clostridium such as Clostridium perfringens and/or Clostridium botulinum and/or Clostridium difficile; from Escherichia such as Escherichia coli (e. g. ETEC, EPEC, EHEC, EAEC, EIEC); from Yersinia such as Yersinia enterocolitica; from Vibrio such as Vibrio cholerae and/or Vibrio parahemolyticus; from Listeria such as Listeria monocytogenes; from Aeromonas such as Aeromonas hydrophila; from Salmonella such as Salmonella enteritidis and/or Salmonella typhimurium; from Shigella such as Shigella dysenteriae; from Campylobacter such as Campylobacter jejuni and/or Campylobacter coli; and/or from Plesiomonas such as Plesiomonas shigelloides.

Preferably, the substance derived from a bacterium is derived from a part of the bacterium, such as an epitope and/or a membrane protein.

Advantageously, by using the inventive device a virus or a part of the virus can be detected with high sensitivity by using a detection agent, i. e. a probe molecule that binds specifically to a substance exposed on the surface of the virus.

Particularly preferred, the substance of the at least one virus is selected from an epitope, preferably from an epitope against B-lymphocytes or T-lymphocytes.

More preferably the viral epitope is selected from a spike protein and/or a nucleocapside protein and/or an envelope protein and/or a membrane protein. Even more preferably, the epitope is selected from an S-glykoprotein S1 and/or S2 and/or a nucleocapside protein N1 or N2 and/or an ACE2 receptor binding site and/or a CD26 binding site. In particular, such substances may comprise one or more regions of the S-glykoprotein S1 and/or S2 and/or an ACE2 receptor binding site.

Further preferably, such a substance comprises an epitope against MHCI (major histocompatibility complex I) and/or MHCII (major histocompatibility complex II).

Preferably, a substance, i. e. a protein, comprises an amino acid sequence shearing at least 60 % sequence identity with a sequence selected from SEQ ID NO:1, SEQ ID NO: 2, SEQ ID NO: 3 or SEQ ID NO: 4, more preferably sharing at least 75 % sequence identity with a sequence selected from SEQ ID NO:1, SEQ ID NO: 2, SEQ ID NO: 3 or SEQ ID NO: 4, even more preferably sharing at least 90 % sequence identity with a sequence selected from SEQ ID NO:1, SEQ ID NO: 2, SEQ ID NO: 3 or SEQ ID NO: 4, in particular sharing at least 95 % sequence identity with a sequence selected from SEQ ID NO:1, SEQ ID NO: 2, SEQ ID NO: 3 or SEQ ID NO: 4, mostly preferably sharing at least 98 % sequence identity with a sequence selected from SEQ ID NO:1, SEQ ID NO: 2, SEQ ID NO: 3 or SEQ ID NO: 4.

According to a particularly preferred embodiment, the substance is selected from a protein sharing at least 80 % sequence identity with a sequence selected from SEQ ID NO: 2, SEQ ID NO: 3 or SEQ ID NO: 4, preferably sharing at least 90 % sequence identity with a sequence selected from SEQ ID NO: 2, SEQ ID NO: 3 or SEQ ID NO: 4, more preferably sharing at least 95 % sequence identity with a sequence selected from SEQ ID NO: 2, SEQ ID NO: 3 or SEQ ID NO: 4, even more preferably sharing at least 98 % sequence identity with a sequence selected from SEQ ID NO: 2, SEQ ID NO: 3 or SEQ ID NO: 4, mostly preferably sharing at least 99 % sequence identity with a sequence selected from SEQ ID NO: 2, SEQ ID NO: 3 or SEQ ID NO: 4.

Preferably, such a substance is obtained from a virus selected from coronavirus, in particular from SARS-CoV-2.

As mentioned above, a mass sensitive micro-array comprises a plurality of mass sensitive detector elements. The detector elements may be arranged in form of a matrix on a basis. The matrix may be arranged one-dimensional, which means that the detector elements are arranged in a simple line or two dimensional in rows and lines. In principle, any array-format may be defined to comprise a number mⁿ of detection elements, wherein m an n are natural numbers.

The device may comprise one of such a MSMA in one detection compartment.

In principal, the mass sensitive micro-array can be constructed to comprise a detection compartment which is used to subsequently measure different samples, e. g. purified samples derived from different sources.

According to a preferred embodiment, the device comprises more than one detection compartment, e. g. at least two detection compartments, more preferably at least four detection compartments, even more preferably at least eight detection compartments, in particular at least sixteen detection compartments.

For example, a device might comprise an array of detection compartments, preferably several neighbouring detection compartments each comprising a group, in particular a line of detector elements.

The device may be constructed such that an MSMA is divided into a plurality (at least two) detection compartments. In this embodiment, one or more groups of the detector elements of the MSMA are separated from each other and/or are enclosed by walls which may be placed between the different groups. In other words, the mass sensitive micro-array is constructed such that it comprises more than one detection compartment. Preferably, such walls are made of polymeric substances, such as PDMS (polydimethyl siloxane).

Such detection compartments can be constructed and bonded to the MSMA, so that there are detection compartments on chip not in direct contact with each other. Such detection compartments can be fabricated from soft polymeric materials such as PDMS or SU-8 (epoxy-based photoresist material, Microchem Corp.).

Alternatively or additionally, the device may also comprise more than one MSMA, whereby, preferably the different MSMA are located in separate detection compartments.

Preferably, each of the detection compartments is connected to a microfluidic device, wherein the microfluidic device comprise a number of inlets or reservoirs each of them being connected via a one or more channels (e. g. syringes) to the different detection compartments. Preferably each of the inlets or reservoirs is connected via a separate channel to a different detection compartment to guide different samples into different compartments.

The different samples, e. g. from blood, saliva, urine, cerebrospinal fluid etc. may be transferred form the inlets or reservoirs simultaneously or subsequently by the microfluidic device into the different detection compartments. Preferably, the different samples are transferred simultaneously into the different compartments.

By using separate detection compartments, the device allows for simultaneous measurement of, e. g. samples and reference samples and/or samples to be tested in parallel for difference target substances. E. g. samples obtained from blood may be tested for different antibodies (such as IgG, IgM) in parallel to samples obtained from saliva to be tested for one or more virus/es.

Thus, according to a further preferred embodiment, at least one of the detector elements in a detection compartment may be configured to generate a reference value.

E. g. by applying four detection compartments, the device allows for simultaneous measurement of two samples (such as samples derived, e. g. from blood or saliva) and two reference samples.

A further number of detection compartments, e. g. eight detection compartments, even allows for simultaneous measurement of further samples, i. e. parallel measurement of samples derived, e. g. from urine, cerebrospinal fluid etc. and/or from samples of different subjects.

The samples may be added automatically or manually into a detection compartment (e. g. via the microfluidic device) or onto the detector elements (e. g. using a pipette or multi-pipette, or a syringe).

A typical liquid sample volume used to be transferred onto a resonator element ranges from, e. g. one or more µl up to 1 ml, preferably at least 2 µl and/or up to 500 µl, more preferably at least 10 µl and/or up to 300 µl, even more preferably at least 20 µl and/or up to 200 µl, in particular at least 50 µl and/or up to 100 µl.

It is understood, that in case for a sample from a subject does not exhibit sufficient liquidity, such a sample may be liquidified appropriately, e. g. by diluting the sample in a suitable buffer such as phosphate buffered saline (PBS). A preferred diluted sample volume would still be used within the above given ranges.

Other objects and features of the present invention will become apparent from the following detailed descriptions considered in conjunction with the accompanying drawings. It is to be understood, however, that the drawings are designed solely for the purposes of illustration and not as a definition of the limits of the invention. In the diagrams, like numbers refer to like objects throughout. Objects in the diagrams are not necessarily drawn to scale.
- Fig. 1: depicts a mass sensitive micro-array (MSMA) according to an embodiment of the present invention;
- Fig. 2: shows a schematic picture for explaining an example of the method of the present invention to detect substances of a saliva sample;
- Fig. 3: shows a schematic picture for explaining an example of the method of the present invention to detect substances of a blood sample;
- Fig. 4: shows a schematic picture for explaining binding of a virus to an activated surface of an mass sensitive detector element which may be used in the MSMA of Figure 1;
- Fig. 5: shows a diagram of a measurement using the inventive device.

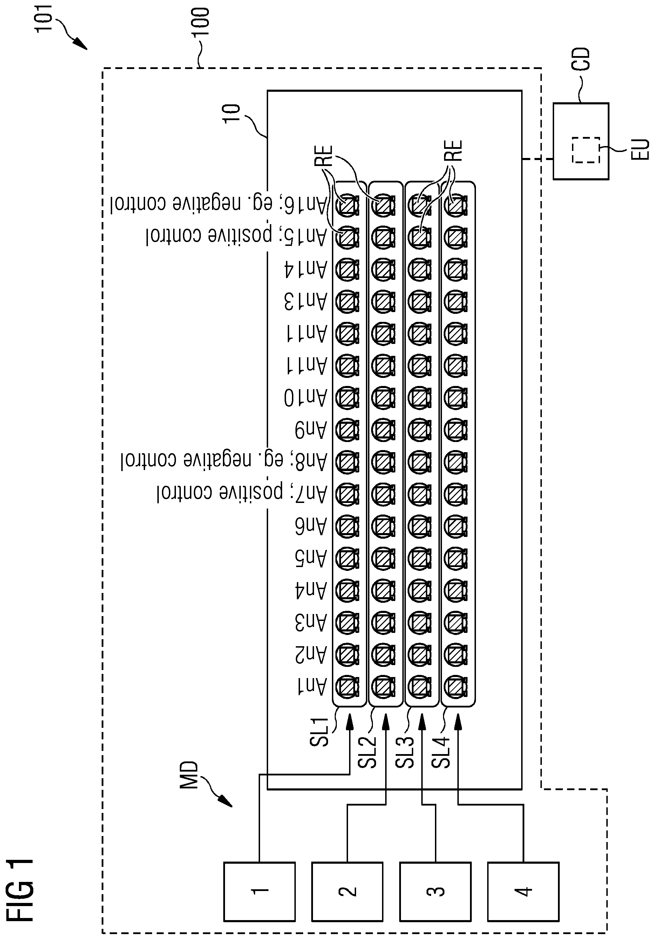

Figure 1 shows a device 100 with a MSMA 10 comprising four detection compartments SL1, SL2, SL3, SL4 being arranged in parallel lines. In the following, those detection compartments SL1, SL2, SL3, SL4 are also named sample lines SL1, SL2, SL3, SL4.

In the MSMA 10 according to Figure 1, each of the four detection compartments SL1, SL2, SL3, SL4 comprise sixteen resonator elements RE (pixels RE), numbered as An1 to An16. Twelve of those sixteen resonator elements RE (the resonator elements An1 to An6 and An9 to An14 in each line) are used for analysis of different substances comprised in a sample. The resonator elements RE are coated with different detection agents, in the present case different substrates to detect substances specific for viruses including substances specific for SARS-CoV-2. Immobilisation of the detection agents on the resonator elements RE may be conducted using a spotting machine, such that different proteins are put onto different resonator elements RE to enable simultaneous detection of different substances from the same sample.

In the case of Figure 1, four of the resonator elements RE of each sample lines SL1, SL2, SL3, SL4 are coated to enable a reference signal, in the present case two resonator elements An7, An15 in each sample line SL1, SL2, SL3, SL4 are used for a positive control (to bind a known substance, e. g. a protein normally abundantly present in e. g. human saliva, such as lysozyme) and further two resonator elements An8, An16 in each sample line SL1, SL2, SL3, SL4 are used as a negative control (not to bind a known substance, e. g. sample not of human origin, such as bovine immunoglobulin).

For a test or measurement, different liquid samples, in the present case samples derived from blood, serum, saliva and urine, may be filled into the different reservoirs 1, 2, 3, 4 of a microfluidic device MD. The samples are transferred via separate syringes of the microfluidic device MD through the sample lines SL1, SL2, SL3 and SL4.

Transfer of the different samples may be induced simultaneously or subsequently; in the present case, the different samples are simultaneously transferred to pass into the different compartments. Upon binding of the substances to be analysed, in the present case a SARS-CoV-2 spike protein, a mass change is detected, thereby inducing a frequency change of the resonator resulting in a change of an electrical signal, which is transmitted to or readout by a control unit CD. The principle of this mechanism is explained e. g. in WO 2004/017063 A1.

The control unit CD comprises an evaluation unit EU. In the present case, the control device CD with the evaluation unit EU is external to the device 100. However, it is in principle possible, that the evaluation unit EU may be comprised in the MSMA 10 itself, for example on the same basis of the resonator elements RE. Binding of substances derived from viruses such as SARS-CoV-2 is calculated in the evaluation unit EU by data-analysis algorithms such that a response of the resonator elements RE coated with specific proteins is compared to a response of positive and negative controls. Calculation can, though, also be performed manually.

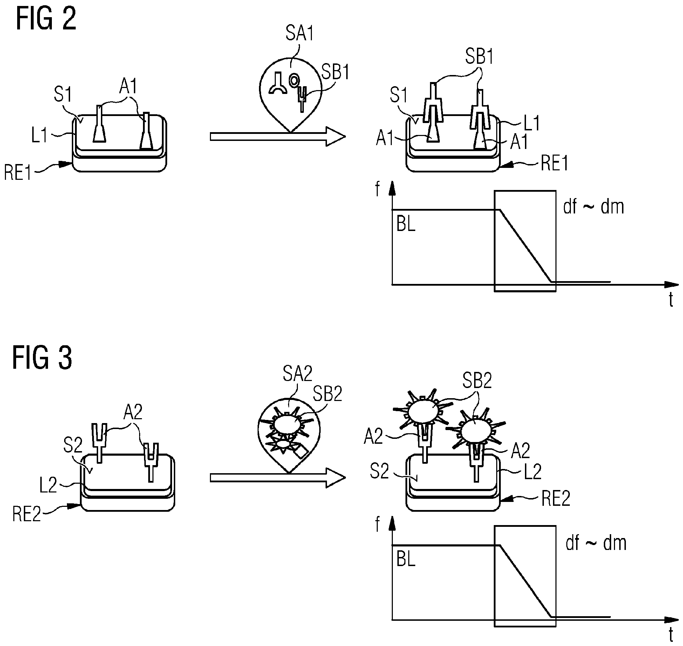

To explain the mechanism of activating a resonator element RE, RE1, RE2 for a specific substance SB, SB1, SB2 it is now referred to Fig. 2, 3 and 4.

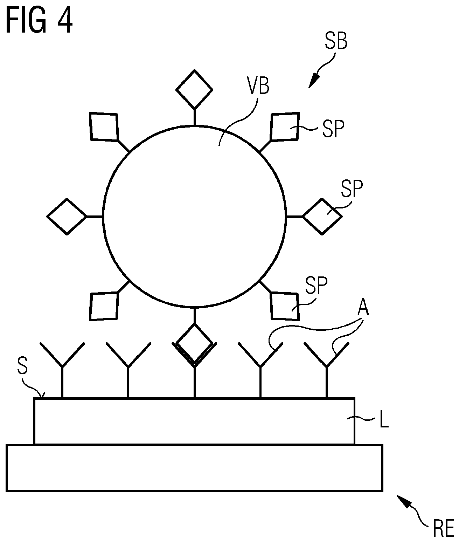

Each of those Figures show a resonator element RE, RE1, RE2 comprising a detection layer L, L1, L2 and a surface S, S1, S2. Detection agents A, A1, A2, in the present case antibodies A, A1, A2, are functionalised onto the surface S, S1, S2 of the detection layer L, L1, L2. Liquid samples SA1, SA2 (not shown in Fig. 4) derived from blood SA1 and saliva SA2 are delivered (e. g. via a microfluidic device MD, as explained in Fig. 1) onto the surface S, S1, S2 of the detection layers L, L1, L2.

Substances SB1, SB2 comprised in the liquid samples SA1, SA2, e. g. antibodies SB1 present in the blood sample SA1 or a virus SB2 present in the saliva sample SA2 are bound onto the surface S1, S2, functionalised with specific antibodies A1, A2, in the present example antibodies A1 recognising antibodies SB1, produced upon SARS-CoV-2 infection and antibodies A2 recognising a spike protein of SARS-Cov-2 SB2.

On the left side of Fig. 2 a resonator element RE1 is shown to comprise a detection layer L1 with a surface S1 which is activated to comprise an immobilised antibody A1. In a next step (depicted by the arrow), a blood sample SA1 to be analysed and comprising different substances (potentially including the target substance SB1 searched for in the sample) is added onto the surface S1 of the detection layer L1. Upon binding of the target substance SB1 to the immobilized antibody A1 (as shown on the right side of Fig. 2), a decrease in the resonance frequency f of the resonator element RE1 is recorded.

The principle of such a measurement curve is shown in the schematic diagram below the resonator element RE1 on the right side of Fig. 2, showing the resonance frequency f of the resonator element RE1 over time t. In the time range prior to the marked area the level of the resonance frequency f is at baseline BL (the level of the surface S1 of the detection layer L1 not comprising any bound substance SB1). Upon sorption of the substance SB1 the resonance frequency f decreases to a lower level. The change df of the resonance frequency is approximately proportional to the change in mass dm of the detector layer L1 due to the sorption of the substance SB1. Additionally, the slope df/dt of the resonance frequency (the speed of the change of resonance frequency) can also be a valuable sensor output. This slope df/dt depends on the binding kinetics of the substance to the substrate, e. g. the time course of the binding of the substance to the substrate.

Fig. 3 shows the same principle and measurement process as described for Fig. 2, with the difference that the detection layer L2 is coated on its surface S2 with the antibody A2. Further, a sample derived from saliva SA2 (instead of blood) is added onto the surface S2 of the detection layer L2. Analogously to Fig. 2, a target substrate SB2 of the saliva sample SA2 binds to the antibody A2, thereby inducing a change of resonance frequency according to the same principle as described in Fig. 2.

Fig. 4 shows a specific example of a resonator element RE which may be used similar to the resonator element of Fig 3, here showing a detection layer L which is coated on its surface S with an antibody A for detection of a virus SB, in particular a SARS-CoV-2 SB, comprised of a virus body VB and spike proteins SP. Binding of the virus SB to the antibodies A occurs via the viral spike proteins SP.

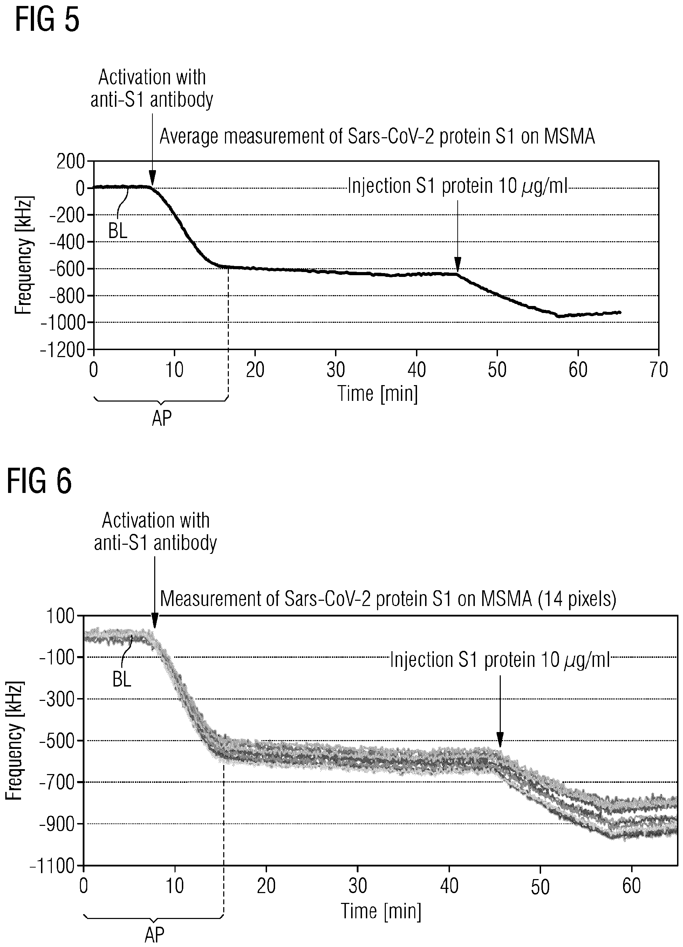

Fig. 5 and 6 show diagrams of measurement curves detected during a measurement process of a sample comprising SARS-CoV-2 virus protein S1 by the MSMA. The curves show a similar principle of the measurement as described in Fig. 2 and 3, however, in contrast to the schematic drawings of Fig. 2 and 3, the diagrams according to Fig. 5 and 6 now show real time measurement curves of the frequency over time.

Fig. 6 shows data of the same measurement as described for Fig. 5. However, whereas Fig. 5 shows the average curve of the fourteen resonator elements, Fig. 6 depicts the signals obtained from each of the fourteen resonator elements.

At time zero to approx. 8 min. Fig. 5 shows the signal of an average baseline BL, in the present case of an average baseline BL of a signal of fourteen resonator elements, not being yet activated with any detection agent. At approx. 8 min., a liquid substrate solution comprising an anti-S1 antibody (dissolved in PBS buffer, as described in Example 1) is added onto the surface of the detection layer. The coating with the liquid substrate is performed by flushing the liquid over the resonator elements using a microfluidic device.

As seen, a decrease of the frequency is observed starting at approx. 8 min. and lasting to approx. 15 min. The phase starting from time zero to approx. 15 min. is named activation phase AP (as depicted in Fig. 5 and 6), as the detection layer of the resonator element does not comprise any substrate. At the end of this activation phase, the resonator element is capable to detect the substance. Accordingly, the level after the activation phase AP is now the new baseline BL for the activated resonator element (corresponding to the baseline of the schematic diagrams of Fig. 2 and 3).

At approx. 45 min. the sample comprising SARS-CoV-2 spike protein S1 at a concentration of 10 µg/ml (in PBS, as explained in Example 1) is injected onto the surface of the resonator elements at continuing flow by using a microfluidic device. Upon binding of the SARS-CoV-2 spike protein S1 to the immobilized anti-S1 antibody, a decrease in frequency is detected by the resonator elements, thereby indicating a change in mass dm of the detector layer due to sorption of the SARS-CoV-2 spike protein S2 (as explained for Fig. 2 to 4 above). The result may be further transferred to a memory.

### Reference signs

- 10: micro sensitive micro-array (MSMA)
- 100: device comprising the MSMA and microfluidic system
- 101: detector kit
- 1, 2, 3, 4: sample reservoirs/inlets
- An1 to An16: resonator elements for analysis
- CD: control system/control unit/control device
- EU: evaluation unit
- MD: microfluidic device
- SL1, SL2, SL3, SL4: detection compartments
- RE, RE1, RE,: resonator element
- SA, SA1, SA2: sample
- SB, SB1, SB2: substrate
- A, A1, A2: detection agent, e. g. a specific antibody
- L, L1, L2: detection layer
- S, S1, S2: surface of the detection layer
- BL: baseline
- f: frequency
- t: time
- df: delta of frequency
- dm: delta of mass
- VB: virus body
- SP: spike protein
- AP: activation phase

### Examples:

### Example 1

### Protocol for Sars-CoV-2 detection on a mass sensitive micro-array (MSMA):

Anti-S1 antibodies against Sars-CoV-2 (Sinobiologicals, Cat.No. 40150) were immobilised on the surface of the resonator elements at a concentration of 5 µg/ml or 10 µg/ml (in PBS) as well as a negative control (antibody against amphetamine, Fitzgerald Scientific, 10-A44F, 10 µg/ml (in PBS)). The coating may be performed manually by spotting/ pipetting the liquid over the whole sensor surface and incubating for up to 30 minutes, or by using an automated spotter machine, which places different antibodies on different sensor pixels, thereby allowing detection of different pathogens from same sample.

Afterwards, Sars-CoV-2 spike protein S1 modified with His-tag (Sinobiologicals, Cat.No. 40591) was passed over the modified resonator surface at continuing flow by using a microfluidic device with a syringe. Sample concentration was 10 µg/ml in PBS. Binding of the Sars-CoV-2 spike protein S1 to the immobilised anti-S1 antibody was monitored by continuous measurement of frequency as depicted in Fig. 5 and 6.

In the present example, the size of the SARS-CoV-2 spike protein S1 was calculated to be around 5.5-6.1 * 10⁶ g/mol, which corresponds to 5.5-6.1 MDa (mega Daltons).

Any sample sources preferably derived from human or animal subjects may be used to conduct the measurement.

In particular, such a sample is used for detection of an infection by a pathogen, preferably by a virus, more preferably by a corona virus, in particular by SARS-COV-2.

Particularly preferred, the method is conducted for the
1. Detection of virus/es from saliva sample;
2. Detection of antibodies from serum sample;
3. In parallel/ sequential detection of saliva and serum sample;

### 1. Detection of virus/es from saliva sample:

Saliva sample can be collected either by passive drooling method, spitting in a tube or using a sample collection swab, in which case the donor or collecting person moves the swab inside donor mouth and cheeks. The sample can also be nasopharyngeal swab. In this case, after collection the collection device is put in a tube containing a suitable buffer (e. g. PBS, pH 7.4, as described above), into which the pathogen from swab is transferred.

In order to conduct the measurement, the surface of the detection layer is coated with antibodies specific to the virus in sample (e. g. by coating an anti-spike S1 and/or S2 antibody (obtained e. g. from Biomedica Medizinprodukte GmbH, Vienna, Austria) against SARS-CoV-2 spike protein S1 / S2) .

The coating can be done either manually by flushing the liquid over the whole sensor surface or by using an automated spotter machine (as described above).
Such an automated spotter machine may also place different antibodies onto each surface of the different detection layers, thereby allowing detection of different pathogens from the same or different samples.

A pre-treatment to break the pathogen (comprised in the sample) into fragments might be implemented. This can be performed by mixing the sample with a buffer solution containing detergents and/or organic solvents. Alternatively or additionally, the sample may be ultrasonicated before being transferred onto the surface of the detection layer.

The liquid sample to comprise the pathogen is then transferred onto the surface of the detection layer, which may be realised by a microfluidic device via i) a syringe, ii) a (multi)pipette or iii) a specific device built for this purpose connected to resonator element (as described above). Consequently, the sample is flown over the activated surface of the detection layer and the pathogen, if present, is bound to the antibodies immobilised on surface.

Positive control (e. g. lysozyme in PBS, pH 7.4) and negative control (e. g. bovine immunoglobulin in PBS, pH 7.4) are conducted in parallel on different resonator elements.

If the pathogen is present in the sample, the frequency of the resonator element will decrease. From this decrease in frequency, presence or absence of the pathogen in question is calculated via a data analysis algorithm.

### 2. Detection of antibodies against bacterial or viral infection in a serum sample:

A blood sample is taken either from a finger tip or from venous blood. The blood is pretreated to remove red blood cells either by microfluidic filtration or by centrifugation to obtain the serum.

The surface of the detection layer is, in this case, coated with a protein from a pathogen, e. g. in case of SARS-CoV-2 nucleocapside proteins N1 and/or N2 (obtained from e. g. Sinobiological).

Alternatively, the protein might be also derived from a spike protein or a membrane/envelope protein or any other protein of SARS-CoV-2.

Nucleocapside N1 and N2 proteins are immobilised on the resonator surface, preferably by spotter machine.

Positive and negative controls may be included as described above.

The serum sample is then flushed over the surface of the different detection layers via a microfluidic device connected to the MSMA. Binding of antibodies contained in the serum sample is detected by a decrease in frequency of the resonator element.

Binding of antibodies is manually calculated or by data-analysis algorithms.

Although the present invention has been disclosed in the form of preferred embodiments and variations thereon, it will be understood that numerous additional modifications and variations could be made thereto without departing from the scope of the invention. For the sake of clarity, it is to be understood that the use of "a" or "an" throughout this application does not exclude a plurality, and "comprising" does not exclude other steps or elements. Also, the mention of a "unit" does not preclude the use of more than one unit.

<212> PRT
<213> SARS Coronavirus

SEQ ID NO 1
<223> ACE2 Receptor Binding Protein (Human)












SEQ ID NO 2
<223> SARS-CoV-2 Spike S1 Protein (RBD)
RVQPTESIVRFPNITNLCPFGEVFNATRFASVYAWNRKRISNCVADYSVLYNSASFSTFKCYGVSPTKLNDLCFTNV
YADSFVIRGDEVRQIAPGQTGKIADYNYKLPDDFTGCVIAWNSNNLDSKVGGNYNYLYRLFRKSNLKPFERDISTEI
YQAGSTPCNGVEGFNCYFPLQSYGFQPTNGVGYQPYRVVVLSFELLHAPATVCGPKKSTNLVKNKCVNF

SEQ ID 3
<223> SARS-CoV-2 Spike Protein S1 Receptor-Binding Domain
NITNLCPFGEVFNATRFASVYAWNRKRISNCVADYSVLYNSASFSTFKCYGVSPTKLNDLCFTNVYADSFVIRGDEV
RQIAPGQTGKIADYNYKLPDDFTGCVIAWNSNNLDSKVGGNYNYLYRLFRKSNLKPFERDISTEIYQAGSTPCNGVE
GFNCYFPLQSYGFQPTNGVGYQPYRVVVLSFELLHAPATV

SEQ ID NO 4
<223> SARS-CoV-2 Nucleocapside
MSDNGPQNQRNAPRITFGGPSDSTGSNQNGERSGARSKQRRPQGLPNNTASWFTALTQHGKEDLKFPRGQGVPINTN
SSPDDQIGYYRRATRRIRGGDGKMKDLSPRWYFYYLGTGPEAGLPYGANKDGIIWVATEGALNTPKDHIGTRNPANN
AAIVLQLPQGTTLPKGFYAEGSRGGSQASSRSSSRSRNSSRNSTPGSSRGTSPARMAGNGGDAALALLLLDRLNQLE
SKMSGKGQQQQGQTVTKKSAAEASKKPRQKRTATKAYNVTQAFGRRGPEQTQGNFGDQELIRQGTDYKHWPQIAQFA
PSASAFFGMSRIGMEVTPSGTWLTYTGAIKLDDKDPNFKDQVILLNKHIDAYKTFPPTEPKKDKKKKADETQALPQR
QKKQQTVTLLPAADLDDFSKQLQQSMSSADSTQA

## Claims

1. A device (100) for detecting at least one substance (SB, SB1, SB2) of a sample (SA1, SA2), preferably in a liquid (SA1, SA2), comprising:
- a mass sensitive micro-array (10) with a plurality of mass sensitive detector elements (RE, RE1, RE2) each comprising a detection layer (L, L1, L2) with a surface section (S1, S2),
- wherein the detection layer (L, L1, L2) of at least one of the mass sensitive detector elements (RE, RE1, RE2) is activated for sorption of the at least one substance (SB, SB1, SB2) of a sample (SA1, SA2), which is preferably obtained from a subject, preferably a human or an animal, more preferably a human, in particular wherein the sample (SA1, SA2) is chosen from blood, plasma, serum, saliva, cerebrospinal fluid, sweat, tears, faeces, vomit, nasopharyngeal swab, tracheal aspirate and/or urine.

2. A device according to claim 1, wherein the mass sensitive micro-array (10) comprises a resonator array (10) with a plurality of film bulk acoustic resonator elements (RE, RE1, RE2), preferably implemented as solidly mounted resonator elements.

3. A device according to claim 1 or 2, wherein each of the mass sensitive detector elements (RE, RE1, RE2) in the mass sensitive micro-array (10) serves to detect a specific substance (SB, SB1, SB2).

4. A device according to any of the preceding claims, comprising an evaluation unit (EU),
- wherein the mass sensitive detector elements (RE, RE1, RE2) are connected to the evaluation unit (EU) to transfer at least one sample value to the evaluation unit (EU),
- wherein the evaluation unit (EU) is constructed such that it quantifies the at least one sample value and optionally compares the at least one sample value with an at least one reference value.

5. A device according to any of the preceding claims, wherein the surface (S, S1, S2) or a section of a surface (S, S1, S2) of the detection layer (L, L1, L2) is coated with a surface coating comprising detection agent (A, A1, A2), preferably by a molecule to comprise biological material, a biologically derived material or a biomimetic.

6. A device according to claim 5, wherein the surface coating of the detection layer (L, L1, L2) comprises a detection agent (A, A1, A2) selected from:
- at least one protein,
and/or
- at least one engineered protein,
and/or
- at least one antibody and/or part/s of an at least one antibody, wherein the at least one antibody and/or part/s of an antibody are preferably chosen to bind to at least one pathogen and/or at least one protein of the at least one pathogen and/or at least one epitope of the at least one pathogen and/or at least one toxin derived from the at least one pathogen,
and/or
- at least one aptamer,
and/or
- at least one biomimetic.

7. A device according to any of claims 5 and 6, wherein the surface coating comprises at least one protein selected from:
at least one antibody against at least one of a spike protein, a nucleocapside protein, an envelope protein and/or
at least one ACE2 receptor binding protein.

8. A device according to any of the preceding claims, wherein the substance is selected from one or more proteins, DNA, RNA, virus/es or parts of virus/es, bacteria and/or toxin/s.

9. A device according to claim 8, wherein the substance is selected from:
at least one virus, wherein the at least one virus is preferably selected from influenza viruses, in particular from influenza A virus; from gastrointestinal viruses, in particular from rotavirus, norovirus, adenovirus or astrovirus; from respiratory viruses, in particular from human bocavirus 1; from a respiratory syncytial (RS)-virus;
from coronaviruses, in particular from HCoV-HKU1 (human coronavirus HKU1), HCoV-NL63 (human coronavirus NL63), HCoV-OC43 (human coronavirus OC43), HCoV-229E (human coronavirus 229E), SARS-CoV (severe acute respiratory syndrome-related coronavirus), MERS-CoV (middle East respiratory syndrome-related coronavirus) and/or SARS-CoV-2 (severe acute respiratory syndrome-related coronavirus 2).

10. A device according to any of claims 8 or 9, wherein the substance of the at least one virus is selected from an epitope, preferably from an epitope against B-lymphocytes and T-lymphocytes, more preferably from a spike protein, an envelope protein and/or a membrane protein and/or a nucleocapside protein, even more preferably from an S-glykoprotein and/or a nucleocapside protein N1 or N2 and/or an ACE2 binding site and/or a CD26 binding site.

11. A device according to any of the preceding claims, wherein the device, preferably the mass sensitive micro-array (1) comprises at least one detection compartment (SL1, SL2, SL3, SL4), preferably at least two detection compartments, more preferably at least four detection compartments, in particular at least eight detection compartments.

12. A device according to any of the preceding claims, wherein at least one of the detector elements (RE, RE1, RE2) is configured to generate a reference value.

13. Method for the analysis of at least one substance (SB, SB1, SB2) in a sample (SA1, SA2), preferably in a liquid, comprising the steps of
(i) providing a mass sensitive micro-array (10) with a plurality of mass sensitive detector elements (RE, RE1, RE2) each comprising a detection layer (L, L1, L2) with a surface section (S, S1, S2),
(ii) activating the detection layer (L, L1, L2) of at least one of the mass sensitive detector elements (RE, RE1, RE2) for sorption of the at least one substance (SB, SB1, SB2) of the sample (SA1, SA2),
(iii) applying the sample (SA1, SA2) onto the mass sensitive detector elements (RE, RE1, RE2,
wherein the sample (SA1, SA2) is preferably obtained from a subject, more preferably a human or an animal, even more preferably a human, in particular wherein the sample (SA1, SA2) is chosen from blood, plasma, serum, saliva, cerebrospinal fluid, sweat, tears, faeces, vomit, nasopharyngeal swab, tracheal aspirate and/or urine.

14. A method according to claim 13, wherein the method further comprises the steps of
(iv) obtaining at least one sample value from a mass sensitive detector element,
(v) quantifying the at least one sample value,
(vi) optionally comparing the at least one sample value with an at least one reference value.

15. A detector kit (101) comprising at least one device (100) according to any of claims 1 to 12 and comprising a control system (CD), preferably wherein the control system (CD) comprises an evaluation unit (EU) which is constructed such that it quantifies at least one sample value obtained from a mass sensitive detector elements (RE, RE1, RE2) and optionally compares the at least one sample value with an at least one reference value.

16. A detector kit comprising at least one device (100) according to any of claims 1 to 12, preferably a kit according to claim 15, wherein the kit comprises detection agents for detecting in a sample of the subject the presence, preferably the amount, of the at least one substance, particularly preferably wherein the kit comprises detection agents for detecting in a sample of a subject the presence of a virus, mostly preferred the presence of a corona virus.
